# EUROPEAN PATENT APPLICATION

(11) **EP 1 548 636 A1**
(43) Date of publication of application: **29.06.2005**
(21) Application number: 03795226.4
(22) Date of filing: 19.08.2003
(51) Int. Cl.: G06K 19/06, G06K 19/10, G06K 7/12, C09K 11/08

(54) **CIPHER INFORMATION-CONTAINING MATERIAL, ITS IDENTIFYING METHOD, AND ITS IDENTIFYING SYSTEM**

(30) Priority: 19.08.2002 JP 2002237828
(71) Applicant: Plagenom Co., Ltd., Osaka-shi, Osaka 542-0086 (JP)
(72) Inventor: KUSHIDA, Takashi, Ako-shi, Hyogo 678-0223 (JP)
(74) Representative: Viering, Jentschura & Partner
(86) International application number: PCT/JP2003/010430
(87) International publication number: WO 2004/025550

(57) **Abstract**

A material for preventing forgery and false alteration. The material comprises a particulate information presenting substance. The information presenting substance is one or more elements, a compound of two or more elements, or a substance containing the elements or compounds. The fluorescence emitted from the substance is different depending on the production history and cipher information different depending on the production history is related to the substance. Even if the fluorescence from such an information presenting substance is detected by a false third party, the production history of the information presenting substance cannot be grasped from the fluorescence. Therefore the fluorescence of the information presenting substance cannot be reproduced, and a high confidentiality of the cipher information given to the material can be ensured.

## Description

### Field of the Invention

The present invention relates to a cipher information containing material, its identifying method, and its identifying system in which a material such as plastic resin, paint, ink, fiber, paper, or metal is provided with a particulate information presenting substance to contain a specific mode of cipher information.

### Description of the Background Art

There have been a variety of articles including cash cards or credit cards, a various certificates such as passports, policies, and a driver license, and brand products which are tagged with magnetized markings, electromagnetic wave absorbing or reflecting markings, or fluorescence emitting markings upon being exposed to visible light. The markings are examined for judging whether the articles are authentic or not, while any false article is tagged with non or a forgery of the marking.

Also, industrial products and food products are attached at their production step with similar markings or labels for identifying the production site, the distributing route, and other information.

Moreover, such markings or lettering are applied to industrial wastes from plants or their products for ease of recovery or sorting of their materials.

However, such traditional markings may easily be destroyed, removed off, or replaced by a false one by any unauthorized third party.

For eliminating the above drawback, a material is provided with a substance which comprises one or more elements or a compound of the elements while assigned with a mode of cipher information determined depending on the type and content of the substance (referred to as an information presenting substance hereinafter). When the material is exposed to a predetermined wavelength range of electromagnetic wave, its information presenting substance emits a range of fluorescence which is then detected. The result of detection of the fluorescence emitted from the information presenting substance is examined to determine the type and content of the information presenting substance. Consequently, the cipher information can be obtained from the type and content of the information presenting substance.

It is still provable that the fluorescence emitted from the information presenting substance of the material is cleverly detected by any unauthorized third party which can thus measure the type and content of the information presenting substance and reproduction the fluorescence of the information presenting substance. Accordingly, the cipher information carried in the information presenting substance will hardly be ensured in the secrecy. More particularly, upon cleverly measuring the fluorescence emitted from the information presenting substance of the material, the unauthorized third party can grasp the type and content of the information presenting substance of the material. This allows the type and content of the information presenting substance to be applied to any false material which can thus be identified as the authentic material from detecting the fluorescence. In turn, the authentic material may be changed to another material by any unauthorized third party modifying the information presenting substance in the predetermined type and content.

### SUMMARY OF THE INVENTION

The present invention has been developed in view of the above drawbacks and its object is to provide a cipher information containing material, its identifying method, and its identifying system in which the cipher information assigned to the material can be protected at higher secrecy by not allowing any unauthorized third party to reproduce the fluorescence from its information presenting substance, thus preventing forgery and false alteration of the material.

The present invention points out that the particulate information presenting substance produces an emission of fluorescence depending on the production history and is thus featured by the information presenting substance produced by the production history which corresponds to a specified cipher information thus to be assigned with the cipher information and applied to the material, whereby the material can carry the cipher information.

For achievement of the foregoing object of the present invention, a cipher information containing material provided with a particular information presenting substance is characterized in that the information presenting substance comprises one or more elements which emit a different range of fluorescence depending on their production history when it is exposed to a predetermined wavelength range of electromagnetic wave, a compound of two or more of the elements, or a substance containing the elements or the compound, and in that the information presenting substance is assigned with a mode of cipher information since having been produced by the production history corresponding to the cipher information.

In this application, the term "material" includes a component used for fabricating a product and a product produced from the components. Also, the term "cipher information" includes a set of symbols assembled intricately and submitted to those which may concern as well as a character, a figure, a symbol, and their combination. The term "production history" includes methods of producing the information presenting substance and conditions in the production such as baking temperature and baking time.

Even if the fluorescence emitted from the information presenting substance is detected by any unauthorized third party, it can hardly disclose the production history form the fluorescence which is varied almost indefinitely. Therefore, the unauthorized third party will certainly fail to produce the information presenting substance without using the same production history and reproduce the fluorescence emitted from the information presenting substance. The cipher information can thus be protected at higher secrecy.

The information presenting substance may preferably be arranged to produce one or more line spectrum depending on the production history when exposed to the specified wavelength range of electromagnetic wave.

As the line spectrum is narrow with the intensity of the fluorescence remaining high, the fluorescence from the information presenting substance can be detected at higher accuracy.

The information presenting substance may preferably include the transition element of incomplete 3d shell and/or the transition element of incomplete 4f shell.

Since the information presenting substance exhibits a line spectrum, its fluorescence can be detected at a higher accuracy.

The information presenting substance may preferably range from 1 nm to 1000 nm in the particle diameter.

As the emission of fluorescence from the information presenting substance depends on the production history, the cipher information carried in the material can be identified easily and assuredly.

The information presenting substance may preferably be coated at the outer surface with another substance.

As a result, the particle size and structure of the information presenting substance can favorably be fixed thus to improve the efficiency of the emission of fluorescence. As its information presenting substance is readily dissolved in a specific solvent, the material can be closely in affinity with neighbor materials.

According to the present invention, a method of identifying the cipher information containing material is provided comprising the steps of irradiating a predetermined wavelength range of electromagnetic wave to the cipher information containing material, detecting an emission of fluorescence produced by the information presenting substance in response to the irradiation, specifying from the result of measurement of the fluorescence emitted from the information presenting substance the cipher information determined by the production history of the information presenting substance, and identifying the cipher information containing material from the cipher information.

Therefore, the cipher information assigned to the information presenting substance can readily be extracted thus allowing the material to be identified with ease and certainty.

According to the present invention, an identifying system for identifying the cipher information containing material is provided comprising a detecting means for irradiating a predetermined wavelength range of electromagnetic wave to the cipher information containing material and detecting an emission of fluorescence produced by the information presenting substance in response to the irradiation, a specifying means for detecting from the result of detection of the fluorescence emitted from the information presenting substance the cipher information determined by the production history of the information presenting substance, and an identifying means for identifying the cipher information containing material from the above cipher information.

Hence, the cipher information assigned to the information presenting substance can readily be extracted thus allowing the material to be identified with ease and certainty.

### BRIEF DESCRIPTION OF THE DRAWINGS

Fig. 1A is a spectrum analysis diagram of the fluorescence emitted from an information representing substance made of yttrium oxide including europium at 750 °C of the baking temperature;
Fig. 1B is a spectrum analysis diagram of the fluorescence emitted from the information representing substance made of yttrium oxide including europium at 500 °C of the baking temperature;
Fig. 2A is a spectrum analysis diagram of the fluorescence emitted from the information representing substance made of yttrium oxide including europium by a gas solidification method;
Fig. 2B is a spectrum analysis diagram of the fluorescence emitted from the information representing substance made of yttrium oxide including europium by a sol/gel method;
Fig. 3 is a diagram showing the relationship between the peak intensity ratio of the fluorescence emitted from the information representing substance made of yttrium oxide including europium and its baking temperature;
Fig. 4 is a diagram showing the relationship between the peak intensity ratio of the fluorescence emitted from the information representing substance made of yttrium oxide including europium and its particle diameter;
Fig. 5 is a schematic view of an identifying system for identifying a cipher information containing material showing one embodiment of the present invention;
Fig. 6 is a schematic view of the hardware arrangement of a detecting apparatus shown in Fig. 5;
Fig. 7 is a schematic view of the hardware arrangement of a computer shown in Fig. 5;
Fig. 8 is a flowchart showing an action of the identifying system shown in Fig. 5;
Fig. 9 is a spectrum analysis diagram of the fluorescence emitted at the room temperature from CdS particles including europium made by a colloid method; and
Fig. 10 is a spectrum analysis diagram similar to Fig. 9, where the fluorescence is 90 degrees out of phase from the exciting light modified in the intensity.

### DESCRIPTION OF THE PREFERRED EMBODIMENTS

Some embodiments of the present will be described below.

### (Cipher information containing material)

A cipher information containing material (A) is the cipher information containing material provided with the particulate information presenting substance. This information presenting substance comprises one or more elements, a compound of two or more elements, or a substance containing the elements or compound, which emits different fluorescence depending on the production history when is exposed to a particular wavelength range of electromagnetic waves. Since the information presenting substance is produced by a production history which is assigned with the specific cipher information, it can be identified by the cipher information.

The cipher information containing material (A) will now be described in more detail.

The material (A) is based on plastic resin, paint, ink, paper, fiber, or metal. The material (A) canbeusedinavarietyofproducts. For example, the material (A) may be provided in the form of house-hold electric appliances, garments, ornaments, bags, shoes, accessories, watches, rings, cloths, stationery, kitchenware, interior products, paints, hanging scrolls, and any other marketable commodities. The material (A) may also be provided with any of natural products, which can be marketed for sale, including meat, vegetables, mountain plants, fish, beverages, processed foods, and medical products. The material (A) may further be used for credits, bonds, certificates, notes, coins, passports, driver licenses, health insurance cards, checks, stocks, and others which are marketed or transferred with their public or private credibility in common societies. The material (A) may be used with packaging materials (inner boxes, outer boxes, manuals, wrappings, carrying bags, card boards, foamed styrol, plastic resin containers) for packaging the above mentioned products or any other labels and tags.

When exposed to a band of electromagnetic waves such as predetermined ultraviolet light, the information presenting substance in the material emits a range of fluorescence corresponding to the wavelength. As the ions in the information presenting substance are excited by the electromagnetic waves such as ultraviolet light, their energy can shift from the fundamental level to a higher level and then to a lower level thus emitting a range of predetermined fluorescence.

It is known that the information presenting substance emits different ranges of fluorescence depending on the type and content. The present invention has been developed focusing on the fact that the emission of fluorescence is differentiated by the production history of the information presenting substance in the material. The information presenting substance which is uniform in the type and content can emit a specified range of fluorescence when it production history (a method or a condition of the production) is varied. More particularly, its fluorescence can be emitted in different spectrums but not the same wavelength.

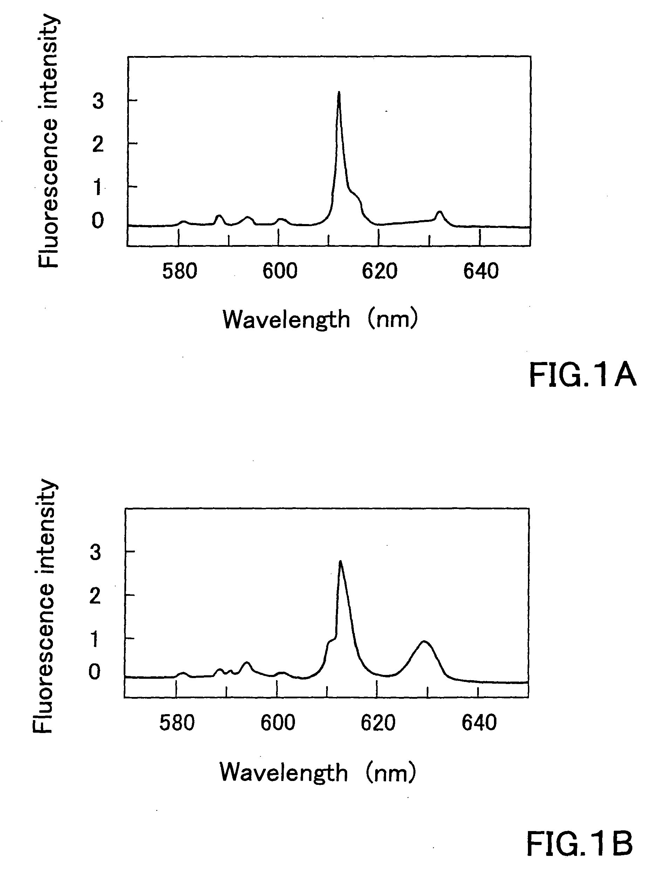

For example, Fig. 1 illustrates two spectrum profiles of fluorescence produced by an information presenting substance made of yttrium oxide (Y₂O₃) containing trivalent europium ions (Eu³⁺); (A) when baked at 750 °C and (B) when baked at 500 °C. It is apparent from Fig. 1 that as the baking temperature is varied, the spectrum of fluorescence slightly changes at 612 nm and 628 nm. Particularly at 628 nm, the fluorescence is significantlyvaried in the intensity. It is thus proved that the emission of fluorescence changes as the baking temperature is varied.

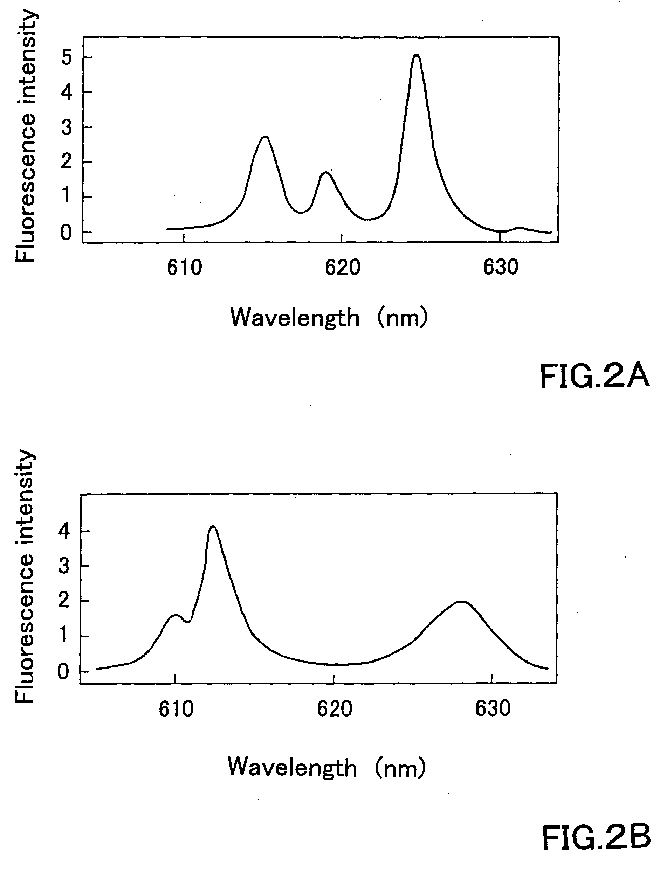

Fig. 2 illustrates two spectrum profiles of fluorescence produced by another information presenting substance made of yttrium oxide (Y₂O₃) containing europium ions (Eu³⁺); (A) when produced by a gas solidifying method and (B) when produced by a sol/gelmethod. It is also apparent from Fig. 2 that as the producing method is varied, the spectrum of fluorescence significantly changes at 612 nm and 628 nm. It is thus proved that the emission of fluorescence changes as the producing method is varied.

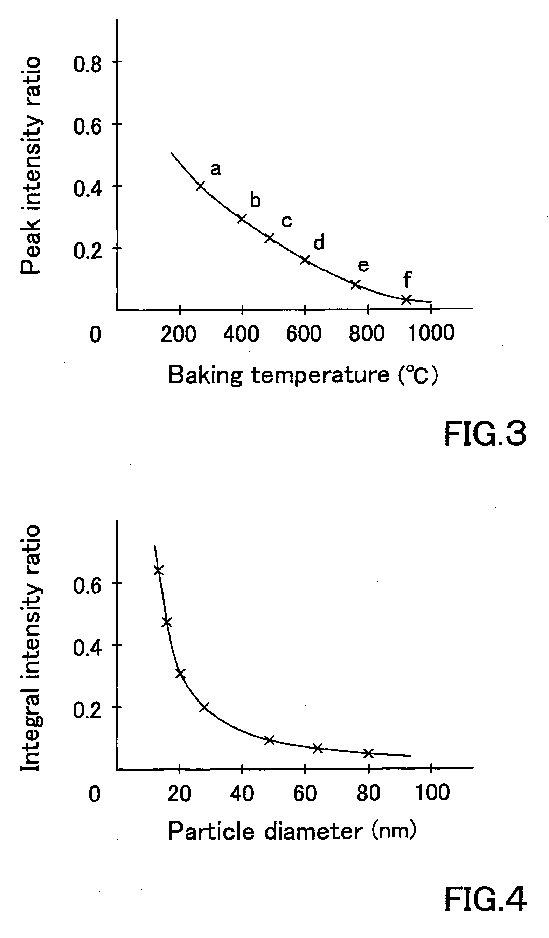

Fig. 3 illustrates a spectrum profile of fluorescence produced by an information presenting substance made of yttrium oxide (Y₂O₃) containing 2 mol% of europium ions (Eu³⁺), where the intensity ratio at the peak between 628 nm and 612 nm of the fluorescence is plotted with different degrees of the baking temperature (equal in the baking time) of the information presenting substance (Y₂O₃: Eu³⁺). It is then apparent from Fig. 3 that as the baking temperature (a condition in the method) is continuously varied, the spectrum of fluorescence emitted from the information presenting substance (Y₂O₃: Eu³⁺) continuously changes. While the baking time remains uniform in the profile of Fig. 3, the emission of fluorescence from the information presenting substance (Y₂O₃: Eu³⁺) may change with a variation in the baking time.

Fig. 4 illustrates a spectrum profile of fluorescence where the integral intensity ratio at the peak between 628 nm and 612 nm of the fluorescence is plotted with different diameters of particles of the information presenting substance (Y₂O₃: Eu³⁺). It is also apparent from Fig. 4 that as the diameter of its particles is continuously varied, the integral intensity ratio of fluorescence emitted from the information presenting substance (Y₂O₃: Eu³⁺) continuously changes. In addition, it is found that the smaller the diameter of the particles, the greater the integral intensity ratio of the fluorescence can increase. It is further proved that the damping constant of the intensity of the fluorescence continuously changes as the diameter of the particles is continuously varied.

As the materials (A) emit different ranges of fluorescence from their different information presenting substances which are varied in the production history and applied to their base materials, they can be assigned with different cipher information respectively.

In general, when the material (A) is provided with an information presenting substance which is assigned with a cipher information depending on the type and content, its emitting fluorescence can easily be intercepted and analyzed by any unauthorized third party to determine the type and content of the information presenting substance. This permits the unauthorized third party to produce a false material provided with the information presenting substance which is identical in the type and content to that of the material (A) for emitting the same range of fluorescence as of the material (A) or to illegally modify the material (A) through providing the information presenting substance which is different in the type and/or content.

On the contrary, when the material (A) is provided with the information presenting substance which is assigned with a particular cipher information depending on its production history, its emitting fluorescence can hardlybe examined by any unauthorized third party trying to find the cipher information because the production history of the information presenting substance has unlimited variations. In practice, the material (A) can rarely be produced by such an unauthorized third party and its emitting fluorescence will never be reproduced. Accordingly, the cipher information in the material (A) can be protected at higher secrecy. No unauthorized third party can be permitted to produce a false material provided with the information presenting substance which is identical in the type and content to that of the material (A) for emitting the same range of fluorescence as of the material (A) or to illegally modify the material (A) through providing the information presenting substance which is different in the type and/or content. Therefore, it is capable of preventing forgery and false alteration.

The information presenting substance in the material (A) is preferably produced from one or more elements which are rarely found in commonly available products.

The preferred element not found in common products may be selected from a series of elements at the atomic number from 31 to 88 or namely lanthanide or more particularly neodymium (Nd), samarium (Sm), europium (Eu), gadolinium (Gd), terbium (Tb), Holmium (Ho), and their combination. Those preferred elements are rarely found in the commonly available materials such as resin, paint, ink, paper, fiber, and metal and their spectrum can easily be measured and analyzed. Also, those elements are inexpensive, sanitary, and commercially available in the form of oxides.

Because of their fluorescence has a narrow wavelength range (as a line) in the spectrum ranging from infrared light to ultraviolet light, one or two elements can be used to construct the information presenting substance.

The element or compound which emits a narrow wavelength range of fluorescence is selected from crystal provided with the transition element of incomplete 3d shell and/or the transition element of incomplete 4f shell, glass provided with the transition element of incomplete 3d shell and/or the transition element of incomplete 4f shell, and complex based on the transition element with the incomplete 3d shell and/or the transition element of incomplete 4f shell. When exposed to a predetermined wavelength range of electromagnetic wave or preferably a range of electromagnetic wave from infrared light to ultraviolet light or more preferably a range of electromagnetic wave from visible light to far infrared light, the information presenting substance emits a narrow wavelength range (as a line) of fluorescence at a higher level of the intensity which corresponds to the range of the electromagnetic wave and can thus be detected at a higher accuracy.

The information presenting substance may comprise a base material provided with a preferred transition element such as yttrium oxide (Y₂O₃) containing the aforementioned europium ions (Eu³⁺). The base material is preferably selected from, but not limited to, oxides, sulfides, nitrides, hydroxides, halides, and other non-organic crystalline material. Alternatively, the base material may be selected from organic, organic/non-organic hybrid compounds, mixture crystalline, amorphous, and glass. For example, the transition element may be contained in a chemically coupling form such as chelete compound, substituted in its crystalline grating with other atoms or ions, inserted in a crystalline grating, or embedded in voids in glass.

When the information presenting substance is made of a non-organic base, it becomes highly stable and when heated to as a high temperature as 1000 °C, can hardly be fractured but remaining in the material (A). As a result, the material (A) can be detected from its emission of fluorescence even when it has been incinerated and disposed thereafter. When the material (A) is made of a liquid form such as water soluble agent solution, organic solvent solution, or turbid liquid, its information presenting substance can remain chemically unchanged and emit a range of fluorescence. Accordingly, the information presenting substance may be applied to such as a liquid waste produced by an industrial plant as well as the commercially available products.

The average diameter of particles of the information presenting substance ranges from 1 nm to 1000 nm, preferably from 1 nm to 500 nm, or more preferably from 1 nm to 100 nm. The particles may be provided in the form of a polymer such as dimer or trimer. As the information presenting substance is decreased in the diameter of particles, a change in its emission of fluorescence can highly be appreciable and thus detected at higher accuracy. The particles of the information presenting substance can be fabricated by any applicable manner such as sol/gel method, colloid method, gas solidification method, gas reactive method, intra-gas evaporating method, sputtering method, glass crystallization method, deposition method, or spraying method.

The information presenting substance may also be coated with a surface decorative agent, such as heavy hydrogen or organic, or covered with any other material than the base material. This permits the information presenting substance to be fixed in the particle form or the structure, thus improving the efficiency of the emission of fluorescence. Also, the information presenting substance can easily be dissolved in a specific solvent and enhanced in the affinity to neighbor materials.

The information presenting substance is applied to a region at the inner or outer side of the material (A). For example, the information presenting substance may be applied to the material (A) by any appropriate manner such as blowing, spraying, coating, absorption, injection, filling, attachment, impregnation, mixture, providing, or chemical coupling (polymerization, bridging, or ion bonding).

More specifically, when the material (A) is made of a plastic resin material, it may be shaped directly after dry blended by a drum tumbler, compounded by an extruder, and compounded or shaped by an internal mixer or heating rolls. Alternatively, the process may be carried out after the material (A) is subjected to master batch processing.

The material (A) may also be impregnated with the information presenting substance with the use of a lubricant, such as fatty amide, fatty metallic salt, or fatty ester, for ensuring uniform distribution and dispersion of the substance.

The amount of the information presenting substance to be applied to the material (A) is controllably determined to such a desired level that the material (A) remains unchanged in the pertinent characteristics in order to give a minimum effect on the external appearance or physical properties. Although the desired amount of the information presenting substance for not largely affecting the pertinent characteristics of the material (A) is variable depending on the type of the material (A), it preferably ranges from 0.1 ppm to 1000 ppm (including 0.1 ppm and 1000 ppm) or more preferably from 0.5 ppm to 200 ppm (including 0.5 ppm and 200 ppm).

It is noted that the amount is not smaller than 0.1 ppm in view of the accuracy of detection which is commonly employed in the relevant field. As the amount is not greater than 1000 ppm, it can hardly affect the external appearance or physical properties of the material (A). Also, the preferable range from 0.5 ppm to 200 ppm is determined for ensuring the reliability of measurement while minimizing the cost of the production. Also, the material (A) can further be protected from declining its pertinent characteristics.

The material (A) may be provided with two or more of the information presenting materials. This allows the material (A) to carry two or more pieces of cipher information or multiple digits of numerical data and thus be identified with a serial number. For example, when the material (A) is provided with six of the different information presenting substances, each expressing 10 grades of the intensity of fluorescence, its emitting fluorescence can be classified into 10⁶ levels of the spectrum. Accordingly when the information presenting substance represents each digit of the number while the intensity of fluorescence from the information presenting substance represents the value in each digit in the number, the material (A) can carry a six-digit number in the decimal notation (000001 to 999999) of the cipher information.

As the cipher information is assigned to the information presenting substance, its variations are determined by methods and conditions of the production history. It is now assumed that two cipher information containing materials (A and B) are produced by heating the information presenting substance (Y₂O₃: Eu³⁺) at two different degrees of the baking temperature (750 °C and 500 °C) respectively. When its emission of fluorescence is detected as shown in Fig. 1A, the information presenting substance can be analyzed by an analyzer that its baking temperature was 750 °C. This results in the identification of the material (A). On the other hand, when measuring the emission of fluorescence of the information presenting substance as shown in Fig. 1B, the analyzer can determine that the information presenting substance was baked at 500 °C. This results in the identification of the material (B). Although any unauthorized third party calculates the type and content of the information presenting substance from its emitting fluorescence shown in Fig. 1A or 1B, it fails to determine the production history of the information presenting substance and will thus produce the information presenting substance with no chance of success.

It is also assumed that the information presenting substance (Y₂O₃: Eu³⁺) was baked at six different degrees of the temperature (300 °C, 400 °C, 500 °C, 600 °C, 800 °C, and 900 °C) to construct six cipher information containing materials (A, B, C, D, E, and F) and its emission of fluorescence exhibits six detections (a, b, c, d, and e) of the peak intensity ratio between 612 nm and 628 nm. Accordingly, from the profile of Fig. 3, the baking temperatures (300 °C, 400 °C, 500 °C, 600 °C, 800 °C, and 900 °C) of the information presenting substance can be determined. As a result, their corresponding cipher information containing materials (A, B, C, D, E, and F) can certainly be identified. In this case, any unauthorized third party fails to find the production history of the information presenting substance while presuming more or less the type and content of the information presenting substance in each material from detection of the peak intensity ratio and will utterly be inhibited from producing the information presenting substance with the same fluorescence.

The production history of the information presenting substance is not limited to the baking temperature and may be any other condition, such as a baking duration of time, or method of the production.

Also, the cipher information is not limited to a particular format but may be selected depending on the material (A). For example, when the material (A) is a credit card or a cash card, its cipher information may be a set of user's data including the ID number. When the material (A) is a check, a bond, or a note, its cipher information may be a set of true/false judging data including the ID number and symbols. When the material (A) is a famous brand product, its cipher information may be a set of manufacturer's data including the serial number, the manufacturer name, the production history, and the materials. When the materials (A) is an industrial product, its cipher information may be a set of manufacturer's data including the serial number, the lot number, the manufacturer name, the production history, and the materials. When the material (A) is a food product label, its cipher information may be a set of food related data including the production site and the production time.

As described, the material (A) provided with the information presenting substance remains rigid because the information presenting substance is chemically stable and very small in the particle size, thus hardly being fractured or peeled off. Also, since the information presenting substance is minimized in the amount with its particle diameter ranging from 1 nm to 1000 nm, it can be identified through physical or chemical analysis only with much difficulty. Moreover, the emission of fluorescence from the information presenting substance is varied depending on the production history including different conditions and methods of the production. This allows the information presenting substance to inhibit any unauthorized third party from identifying the production history from its emission of fluorescence. Accordingly, the material (A) can hardly be forged or modified while permitting no reproduction of the fluorescence. As its information presenting substance is selectively produced in different modes and applicable to almost every commonly available material, the material (A) can carry a desired format of the cipher information at higher secrecy with a variety of products in the multiple fields.

### (Identifying system)

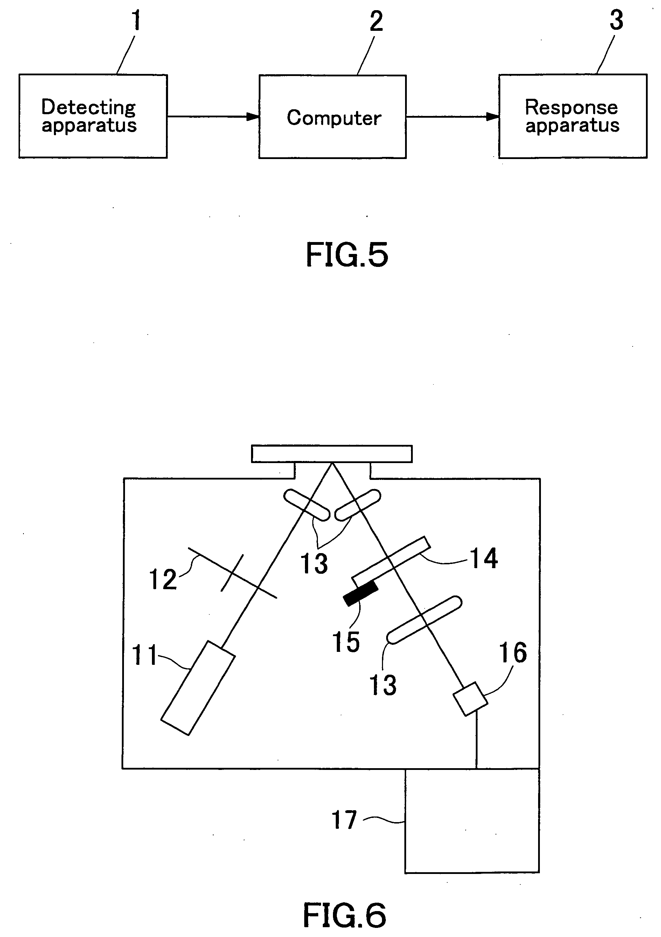

An identifying system for identifying the material (A) will now be described referring to Figs. 5 to 7.

The identifying system comprises, as shown in Fig. 5, a detecting apparatus 1 for detecting the emission of fluorescence from the information presenting substance applied to the material (A), a computer 2 for identifying the material (A) from the result of detection produced by the detecting apparatus 1, and a response apparatus 3 for conducting a predetermined process in response to the result of identification produced by the computer 2. The computer 2 may be connected by a network such as the Internet to the detecting apparatus 1 or the response apparatus 3.

The detecting apparatus 1 is provided for irradiating a predetermined wavelength range of electromagnetic wave to the material (A) and measuring fluorescence emitted from the information presenting substance of the material (A). The result of detection (data of the fluorescent spectrum) from the information presenting substance is expressed in spectrum analysis profiles such as shown in Fig. 1 and 2 where the horizontal axis represents the wavelength of the fluorescence from the information presenting substance while the vertical axis represents the intensity of the fluorescence. The result of detection about the fluorescence is then transferred to the computer 3. The detecting apparatus 1 is preferably implemented by a combination of a semiconductor laser, a CCD, and a light separating system employing a time-division separation or polarizing separation technique.

More specifically, the detecting apparatus 1 comprises, as shown in Fig. 6, an exciting light source 11 such as a germicidal lamp or a semiconductor laser, a chopper 12 for switching on or off the exciting light or modulating the intensity of the exciting light, a lens 13 for shifting the exciting light to parallel light or converging the exciting light, an interference filter 14 for passing desired wavelength of the exciting light, a piezoelectric element 15 for oscillating the angle of the interference filter 14 to modify the wavelengths of the exciting light, a photo-detector 16 for producing a current or voltage corresponding to the intensity of the received light, and a circuit 17 for directing the chopper 12 to extract a desired phase or time component of the exciting light. The detecting apparatus 1 is not limited to the above arrangement and may be implemented by any technology which can detect the emission of fluorescence from the information presenting substance.

The method of the detecting apparatus 1 detecting the emission of fluorescence from the information presenting substance may include steps of switching on and off the electromagnetic wave (or the exciting light) and measuring the fluorescence just after the switching off of the excitation or steps of periodically modifying the intensity of the exciting light and measuring the fluorescence at a desired phase, whereby the fluorescence emitted from the information presenting substance including the ions of the transition element can be detected at higher accuracy. Alternatively, when the fluorescence emitted from the information presenting substance is narrow in the wavelengths (line spectrum in the spectrum), a change in its intensity can be detected with the wavelength of the exciting light or of the fluorescence being modified so as to obtain the spectrum at higher S/N ratios.

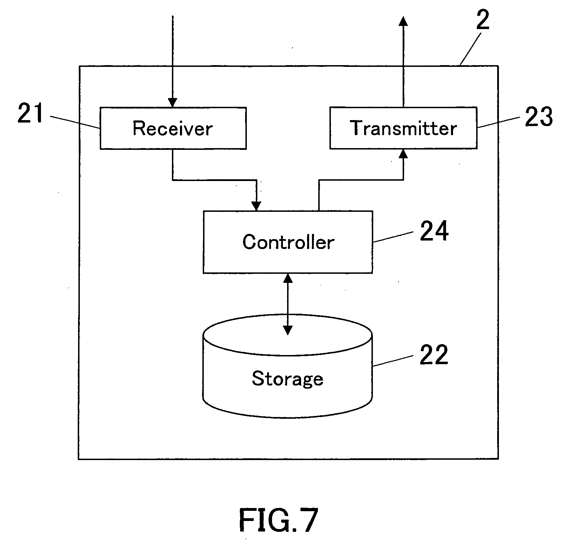

The computer 2 comprises, as shown in Fig. 7, a receiver 21 for receiving from the detecting apparatus 1 the result of detection of the fluorescence emitted by the information representing substance, a reference table storage 22 for storing a reference table to examine the relationship between the information presenting substance and the cipher information, a transmitter 23 for transmitting a data to the response apparatus 3, and a controller 24 for comprehensively controlling the action of each component.

The controller 24 may consist of a central processing unit (CPU) for carrying out data transfer actions, arithmetic operations, and temporal data saving actions. In this embodiment, the controller 24 is arranged to examine the reference table saved in the reference table storage 22 in response to the result of detection received from the receiver 21 for determining the cipher information in the fluorescence emitted by the information presenting substance and identify the material (A) from the cipher information.

The response apparatus 3 is provided for conducting a predetermined response action in response to the identification of the material (A) determined by the computer 2. For example, when the material (A) is any of cash card, credit card, check, bond, note, brand product, and food label, the response apparatus 3 may be accompanied with a monitor and loudspeakers for displaying a true/false data. When the material (A) is an industrial product, the response apparatus 3 may be a sorter for separating the product into different materials. When the material (A) is an ID card for entrance of a specific room, the response apparatus 3 may be a door opening/closing mechanism. When the material (A) is an electronic money or prepaid card, the response apparatus 3 may be an electronic cashing register. When the material (A) is a hospital ID card, the response apparatus 3 may be a monitor or a printer for outputting a carte or medicine data. When the material (A) is a residential ID card, the response apparatus 3 may be a monitor or a printer for outputting a residence certificate or a stamp certificate.

Although the detecting apparatus 1, the computer 2, and the response apparatus 3 are provided separately in this embodiment, they may be modified that at least two are assembled to a single unit.

### (Action of identifying system)

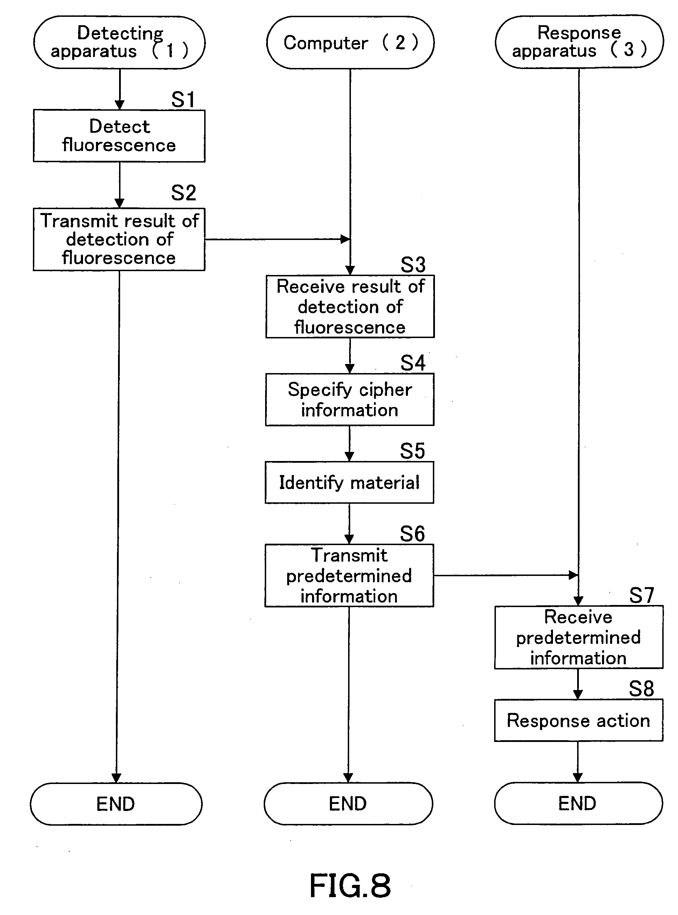

The action of the identifying system will be described referring to a flowchart shown in Fig. 8. In the description and drawings, the step is denoted simply by S.

The action starts with the detecting apparatus 1 irradiating a predetermined wavelength range of electromagnetic wave to the material (A) placed on a predetermined position and measuring the emission of fluorescence from the information presenting substance (S1).

The result of detection of the fluorescence emitted by the information presenting substance is transferred from the detecting apparatus 1 to the computer 2 (S2).

The computer 2 receives at its receiver 21 the result of detection of the fluorescence emitted by the information presenting substance from the detecting apparatus 1 (S3).

The computer 2 then examines at its controller 24 the result of detection of the fluorescence emitted by the information presenting substance received at the receiver 21 through referring the reference table saved in its reference table storage 22 in order to extract the cipher information (S4).

The computer 2 identifies at the controller 24 the material (A) from the cipher information (S5).

The computer 2 transmits the result of identification of the material (A) produced at S5 to the response apparatus 3 (S6).

The response apparatus 3 receives and analyzes the result of identification from the computer 2 to conduct a responding action such as display of a data or sorting of materials (S8).

### (Embodiment 1) Embodiment with information presenting substance of Y₂O₃ including trivalent lanthanide ions

Embodiments of the information presenting substance Y₂O₃ including trivalent lanthanide ions, such as Eu³⁺, Sm³⁺, Tb³⁺, and Er³⁺, were produced by a sol/gel method. Regents for producing were yttrium nitrate-4-hydrate Y(NO)₃:4H₂O, europium acetate-4-hydrate Eu(CH₃CO)₃:4H₂O, samarium acetate-4-hydrate Sm(CH₃COO)₃:4H₂O, terbium acetate-4-hydrate Tb(CH₃COO)₃:4H₂O, erbium acetate-4-hydrate Er(CH₃COO)₃:4H₂O, and sodium carbonate Na₂CO₃. The solvent was distilled water.

Yttrium nitrate and lanthanide acetate were mixed up in a solution and then added with sodium carbonate while being stirred. The mixture was stirred for about 10 minutes and then spun in a centrifugal separator for removing impurities before dried out at about 80 °C for 24 hours. When the mixture was baked at 300 to 900 °C for 1 to 30 minutes, it was turned to the particulate information presenting substance.

The information presenting substance was examined by X-ray diffraction analysis and its average particle size measured ranging from 10 nm to 100 nm. It was also found that the lower the baking temperature or the shorter the baking time, the smaller the particle size of the information presenting substance became.

The information presenting substance was then exposed to the exciting illumination of a xenon lamp or heavy hydrogen lamp and its emitting fluorescence was measured. It was found from the spectrum of the fluorescence and the exciting illumination that Y₂O₃ contained ions of Eu³⁺, Sm³⁺, Tb³⁺, or Er³⁺.

For instance, when its concentration of europium ions was varied from 1 mol% to 10 mol%, the example added with Eu3+ ions was proportionally increased in the intensity of its emitting fluorescence. Although the peak wavelength in the charge transfer band was slightly shifted from 250 nm towards the longer side, the spectrum of visible light remained unchanged.

However, when a particular condition of the production was introduced, the wavelengths at 612 nm and 628 nm of the fluorescence were explicitly shifted as shown in Fig. 1 thus changing significantly the integral intensity ratio. More specifically, it was found that the integral intensity ratio was continuously shifted from 0.1 to 0.9 as the baking temperature was lowered and the braking time was shortened.

Eight test pieces were produced which were identical in the composition and varied in the integral intensity ratio at equal intervals of substantially 0.1 and then exposed to an illumination generated by a green pointer (the second harmonic of a semiconductor neodymium laser) and guided along a fiber optic. The fluorescence emitted from each of the eight testpieces was guided via another fiber optic into a spectroscope for measurement. As a result, the eight testpieces were successfully distinguished from one another at generous S/N ratios.

Meanwhile, a bulk form of Y2O3 crystals including europium ions was subjected to evaporation with the intensified energy of Q switch YAG/Nd laser and cooled down to deposit and solidify a particulate form on a brass bed. It was then found that the particulate form of this testpiece was identical in the composition and the average diameter size to that produced by the sol/gel method but quite different in the spectrum about 620 nm of its emitting fluorescence as well as in the peak wavelength.

Accordingly, it was proved that examples of the information presenting substance produced by different methods or conditions were equal in the composition but significantly different in the spectrum pattern, the peak wavelength, and the intensity ratio.

### (Embodiment 2) Embodiment with information presenting substance of CdS including Eu³⁺

One embodiment of the Cds information presenting substance including trivalent europium ions was produced by a colloid method. Regents for producing were cadmium acetate-2-hydrate Cd(CH₃COO)₂:2H₂O, europium acetate-2-hydrate Eu(CH₃COO)₃:2H₂O, and sodium sulfide-9-hydrate Na₂S:9H₂O. The solvents were dimethyl formamide C₃H₇NO and methyl alcohol CH₃OH. A surface decorative agent of phenyl tio-tri-methyl silane C₆H₅SSi(CH₃)₃ was used for covering the CdS particulate form and fixing the size of its particles.

0.255 mmol of cadmium acetate and 0.025 mmol of europium acetate were dissolved in 100 ml of dimethyl formamide in a flask bottle and cooled down by ice to 0 °C. The mixture was exposed to argon gas for 15 minutes for removing oxygen components from the solution. 0.25 mol of sodium sulfide was mixed with 5 ml of methyl alcohol by an agitator and added to the mixture in the flak bottle. The flask bottle was stirred for one hour and a half by an evaporator. As a result, cadmium acetate and sodium sulfide were reacted together to develop a CdS particular form. The CdS particular form was added with 0.25 mmol of phenyl tio-tri-methyl silane to fix the CdS particulate form at a smaller diameter size. The mixture was then agitated for 30 minutes for stabilization and subjected to evaporation (at 50 °C) in an evaporator. As a result, the mixture was concentrated to 10 ml. The mixture was subjected to a centrifugal action (at 3000 rpm for 30 minutes) for removal of impurities. Then, the mixture was subjected again to evaporation (at 80 °C) in an evaporator for eliminating the solvent and concentrated to 2 ml. After dried out by vacuum drying, the particulate form was deposited on and scraped off from the inner wall of the flask bottle.

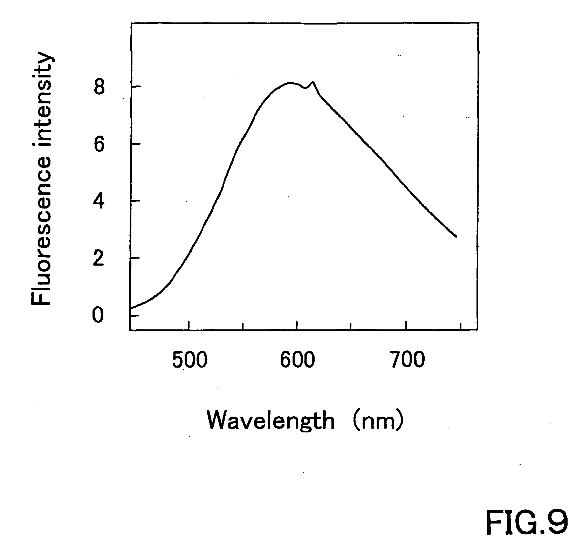

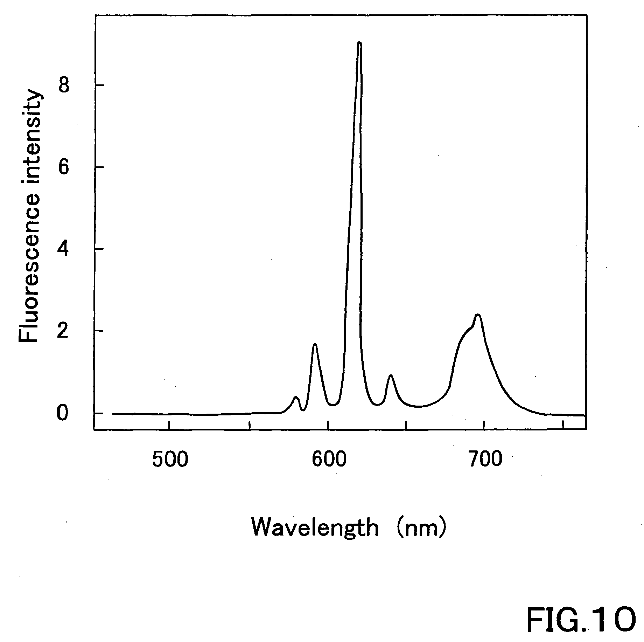

The resultant particles of the information presenting substance were 2.2 nm in average diameter. The spectrum of fluorescence emitted from the information presenting substance by exciting with ultraviolet light had, as shown in Fig. 9, a small line by Eu³⁺ ions at substantially 620 nm overridden on the wide band profile resulted by CdS error. For compensation, the information presenting substance was exposed to an intensity of He-Cd laser light (at 325 nm) modified by a 750 Hz chopper and its emitting fluorescence was detected at 90 degrees out of phase from the exciting laser light. As a result, only a narrow line spectrum by Eu³⁺ ions was observed as shown in Fig. 10.

Accordingly, it was found that even when the fluorescence emitted from the transition element ions was interrupted with any highly intensive external illumination at either the inner or outer side of the material, its spectrum was favorably separated and measured by a desired detecting manner.

### (Embodiment 3)

An embodiment of the information presenting substance Y₂O₃ including trivalent europium was produced by the same sol/gel method as of Embodiment 1 and applied to a 10000 yen note as a marking. Then, the note was cleaned down a number of times using an alcohol saturated cloth and exposed to a green light emitted from a green pointer. The resultant emission of fluorescence from the example was measured by a spectroscope with fiber optic. It was found that a narrow line by Eu³⁺ ions appeared on the spectrum indicating the presence of the marking. Although the narrow line spectrum by Eu³⁺ ions was observed as overridden on the full spectrum of the light, it was successfully extracted from the full spectrum and identified separately by the same manner as of Example 2. It was also found that when this embodiment of the information presenting substance was mixed with a wine in a cup, a narrow line by Eu³⁺ ions was successfully identified from the spectrum of its emitting fluorescence thus indicating the presence of a marking. Moreover, 0.1 mg of this example was mixed with 0.1 ml of ethyl alcohol and a garment cloth and a piece of paper were immersed in the alcohol mixture. The garment cloth and the piece of paper were removed from the alcohol mixture and subjected to volatilization of the volatile alcohol. Then, the spectrum of fluorescence was measured by the same manner. It was found that a narrow line spectrum by Eu³⁺ ions was successfully observed thus indicating the presence of a marking.

## Claims

1. A cipher information containing material provided with a particulate information presenting substance, **characterized in that**:
the information presenting substance comprises one or more elements which emit a different range of fluorescence depending on their production history when is exposed to a specific wavelength range of electromagnetic wave, a compound of two or more of the elements, or a substance containing the elements or the compound, and
the information presenting substance is assigned with a mode of cipher information since having been produced by the production history corresponding to the cipher information.

2. The cipher information containing material according to claim 1, wherein the information presenting substance is arranged to emit one or more line spectrum depending on the production history when exposed to the predetermined wavelength range of electromagnetic wave.

3. The cipher information containing material according to claim 1 or 2, wherein the information presenting substance includes a transition element of incomplete 3d shell and/or a transition element of incomplete 4f shell.

4. The cipher information containing material according to any of claims 1 to 3, wherein the information presenting substance ranges from 1 nm to 1000 nm in the particle diameter.

5. The cipher information containing material according to any of claims 1 to 4, wherein the information presenting substance is coated at the outer surface with another substance.

6. A method of identifying the cipher information containing material comprising the steps of:
irradiating a specified wavelength range of electromagnetic wave to the cipher information containing material and detecting the fluorescence emitted from the information presenting substance in response to the irradiation;
specifying from the result of the fluorescence emitted from the information presenting substance the cipher information determined by the production history of the information presenting substance; and
identifying the cipher information containing material from the specified cipher information.

7. An identifying system for identifying the cipher information containing material, comprising:
a detecting means for irradiating a predetermined wavelength range of electromagnetic wave to the cipher information containing material and detecting the fluorescence emitted from the information presenting substance in response to the irradiation;
a specifying means from the result of the fluorescence emitted from the information presenting substance the cipher information determined by the production history of the information presenting substance; and
an identifying means for identifying the cipher information containing material from the cipher information.
